# EUROPEAN PATENT SPECIFICATION

(11) **EP 2 535 037 B1**
(45) Date of publication and mention of the grant of the patent: **03.12.2014**
(21) Application number: 12166767.9
(22) Date of filing: 04.05.2012
(51) Int. Cl.: A61K 8/04, A61K 8/34, A61K 8/41, A61Q 5/12, A61K 8/19

(54) **Cosmetic composition in a container**
Kosmetische Zusammensetzung in einem Behälter
Composition cosmétique dans un récipient

(30) Priority: 10.05.2011 EP 11165474
(43) Date of publication of application: 19.12.2012
(73) Proprietor: The Procter & Gamble Company, Cincinnati, OH 45202 (US)
(72) Inventor: Loechel, Florian, 35305 Gruenberg-Weitershain (DE)
(74) Representative: Töpert, Verena Clarita

(56) References cited:
- EP-A1- 1 752 128
- EP-B1- 0 462 112
- DE-A1- 10 015 149
- DE-A1- 10 304 721
- US-B1- 6 375 046

## Description

### FIELD OF THE INVENTION

The present invention relates to a cosmetic composition in a container comprising at least one inner bag and an outer container, wherein the outer container encloses the inner bag and is filled with a propellant compressing the inner bag, and a valve mechanism attached to the inner bag moveable between an open position, in which a composition stored in the inner bag is allowed to be discharged by the pressure of the compressed gas in foam form, and a closed position, in which the composition is not allowed to be discharged, wherein the composition within the inner bag comprises 0.1 to 5 % by weight of a cationic surfactant (A), 0.1 to 10 % by weight of a fatty alcohol (B), and 0.1 to 10 % by weight of carbon dioxide.

### BACKGROUND OF THE INVENTION

Hair cosmetic aerosols, particularly foams, for hair conditioning purposes, deliver significant advantages concerning distribution and handling in comparison with emulsions or dispersions. Nevertheless the disadvantages of conventional foams can be the haptic and optical properties. These conventional foams are generally less creamy and rich than emulsions and dispersions and often confer the impression of a lesser achievable effect as compared to a cream or an emulsion. Therefore, many consumers do not use conditioning aerosol foams, especially those with damaged hair, because the optical and haptic properties do not meet the need for creamy and rich textures of consumers having damaged hair.

In order to improve the properties of such foams, it is known to use low-boiling hydrocarbons as propellants, e.g. propane or butane or the like, for foaming the composition as described, e.g. in DE 103 04 721 A1. Such foams are usually offered in aerosol cans, wherein the propellant serves to foam the composition during use if a valve of a dispenser cap is opened. However, such aerosol cans involve the disadvantage that the content may not be sufficiently exhausted. Additionally, such cans have a high sensitivity to correct usage. If they are not held properly upside down, the propellant will evaporate from the can without the product. In order to allow the consumer to dispense the amount indicated on the can, the cans are usually overfilled. As hair care compositions may be expensive, there is a persistent desire to increase the exhaustible amount of the compositions.

A further problem with conventional foams in aerosol cans can lie in the fact that a propellant which may be desirable from the point of how to dispense the foam most economically from the aerosol can, may be undesirable with regard to the properties it confers to the foam. Mixtures of propellants require a multi step filling process or a premixing process for the propellants and can often only deliver a compromise in view of the results regarding quality and texture of the foam, economy of the production process and completeness of exhaustion from the can.

EP 1 342 465 describes the use of carbon dioxide for foaming a liquid hair dye. The liquid hair dye is stored in a bag with a valve attached to, and mixed with the carbon dioxide only when the valve is in an open position.

In order to improve the haptic properties of foam preparations to be used in the field of hair treatment, it has frequently been attempted to give the foam a more mousse-like appearance and a creamier feeling, when applied.

WO 2007/010487 describes an aerosol cream mousse, a method of treating hair with such a mousse and its use. While the haptic properties of such a cream mousse are very good, problems can arise from the choice of propellant. A complete exhaustion of a can containing the composition is often not possible, so overfilling of the can in order to provide the guaranteed amount of cream mousse is necessary. Attempts to achieve a more complete exhaustion of the can by varying the propellant often fail, since a variation of the propellant can change the properties of the cream mousse. Such changes, however, may not be wanted for particular purposes.

It is therefore an object of the present invention to provide a container with at least one inner bag, e.g., a bag-on-valve-system, in an outer container, containing a stable foam, especially a rich, thick and creamy mousse such as an aerosol cream mousse as described in WO 2007/010487, preferably a stable hair care aerosol cream mousse, delivering much more creaminess and richness than conventional foams, which discharge apparatus has an increased level of exhaustion for the mousse from a dispensing device, such as an aerosol can. It has been a further object of the invention to provide a discharge apparatus with a bag-on-valve-system containing a stable foam, especially a rich, thick and creamy mousse such as an aerosol cream mousse as described in WO 2007/010487, which allows for the use of different propellants for the exhaustion of the mousse from the bag and the generation of a creamy foam.

### SUMMARY OF THE INVENTION

This object is solved by a cosmetic composition in a container, having the features of claim 1. Further advantageous embodiments are given in the dependent claims.

It is an aspect of the invention to provide an improved combination of a cosmetic composition and a container in which the cosmetic composition is stored in and discharged from, which has the advantages as described above. The cosmetic compositions are generally discharged as a foam, i.e. as a two-phase composition having a liquid phase as the continuous phase and a gas phase as the discontinuous phase. While the advantages of the invention can be beneficial to any type of foamed preparation for hair treatment, it is especially advantageous for those foams, which have improved haptic properties resulting in a perception of the foam as, e.g., being richer or creamier. Often, such perceptions are associated with a certain viscosity of the liquid phase, or a certain amount of propellant in the composition to be foamed. While the invention is directed to all types of foams, it is very beneficial for foams which can be characterized by the term "mousse", meaning a creamy, rich foam with a character having the lightness of a foam together with the richness and feel of a cream in varying shares.

In the following text the terms "foam" and "mousse" are used interchangeable, apart from those passages where it is especially indicated or clearly understandable that interchanging the terms would not make sense or result in a technically meaningful result.

The basic idea of the present invention is to provide a possibility to offer a foam, especially a creamy aerosol mousse, in a form, where a maximum of exhaustion from an aerosol driven container is possible, but the nature and the features of the foam are not limited by the necessity to use a certain propellant or a combination of propellants only to achieve good exhaustion. The present invention allows for a free choice of propellants responsible for the texture and appearance of the mousse, while still a satisfying exhaustion of the foam, which can be complete or almost complete, is possible. It is even possible according to the present invention to use a combination of propellants which is incompatible during storage, but provides beneficial properties to foam, especially to a mousse product, after mixing for at least a short amount of time.

To achieve the above mentioned advantages, a cosmetic composition is provided which is contained in a flexible bag with a valve, where the composition in the bag contains at least one propellant, preferably at least carbon dioxide and can be discharged from the bag as foam, especially as a mousse. The bag containing the composition can be combined with any type of container which can be filled with any type of propellant in order to achieve a maximum exhaustion of the composition from the bag, while still being able to design the foam resulting from a discharge of the cosmetic composition with regard to propellant depending properties with a maximum degree of freedom.

When the bag is filled with the cosmetic composition and the at least one propellant, it can be inserted into an aerosol can, and the remaining interior space in the aerosol can is then filled with a propellant, such as a compressed gas, e.g. nitrogen, or compressed air, or any other desired propellant. The valve of the bag can serve for sealing the bag as well as the can. For this purpose, two sealings can be provided. A first or outer sealing can serve to seal the space between the can and the bag. A second, or inner sealing can serve to seal the valve of the bag. A dispenser cap is favourably attached to the valve. If the dispenser cap is agitated; the valve moves into its open position and any compressed gas between the can and the bag causes the mousse to escape from the bag and to be discharged through the dispenser cap. The propellant contained in the cosmetic composition in the bag then, is responsible for the foaming of the composition after discharge from the container. It is, however, also possible that the foaming process results from a mixture of propellants, at least one of which is stored as part of the cosmetic composition inside the bag and at least another one of which is stored outside of the bag and thus does not form part of the cosmetic composition stored inside the bag.

Particularly, foaming the composition with a propellant comprising carbon dioxide or consisting essentially entirely of carbon dioxide, results in a stable mousse, which is very smooth and dense and has a consistency and look resembling whipped cream.

### BRIEF DESCRIPTION OF THE DRAWINGS

Fig. 1 is a schematic cross-sectional view of an embodiment of the container described as a part of the present invention;
Fig. 2 is a side view of the inner bag of the container with a valve mechanism attached, and
Fig. 3 is a front view of the inner bag of the container with a valve mechanism attached.

### DETAILED DESCRIPTION OF THE INVENTION

The present invention is described in detail below. Where applicable, the description is made with reference to the diagrams. It should be noted that the same symbols in each diagram represent identical or equivalent constituent elements.

The invention relates to a cosmetic composition in a container comprising at least one inner bag and an outer container, wherein the outer container encloses the inner bag and is filled with a propellant compressing the inner bag; and a valve mechanism attached to the inner bag moveable between an open position, in which a composition stored in the inner bag is allowed to be discharged by the pressure of the compressed gas as a foam, and a closed position, in which the composition is not allowed to be discharged, wherein the composition within the inner bag comprises
a) 0.1 to 5 % by weight of a cationic surfactant(A),
b) 0.1 to 10 % by weight of a fatty alcohol (B), and
c) 0.1 to 10 % by weight of carbon dioxide.

The invention further relates to the use of a container comprising at least one inner bag and an outer container, wherein the outer container encloses the inner bag and is filled with a propellant compressing the inner bag; and a valve mechanism attached to the inner bag moveable between an open position, in which a cosmetic composition stored in the inner bag is allowed to be discharged by the pressure of the compressed gas as a foam, and a closed position, in which the composition is not allowed to be discharged, for discharging a cosmetic composition comprising 0.1 to 5 % by weight of a cationic surfactant(A), 0.1 to 10 % by weight of a fatty alcohol (B), and 0.1 to 10 % by weight of carbon dioxide.

The cosmetic composition is based on the combination of component (A) a cationic surfactant, which is generally a quaternary ammonium compound such as e. g. ditallow dimethyl ammonium chloride, (B) a fatty alcohol, such as e. g. cetyl and stearyl alcohol, and carbon dioxide.

The essential ingredients as well as a variety, but non-exclusive, list of preferred and optional ingredients are described below.

### CATIONIC SURFACTANTS

Cationic surfactants preferably used in the composition of the present invention, contain amino or quaternary ammonium moieties. Cationic surfactants among those useful herein are disclosed in the following documents, all incorporated by reference herein: M. C. Publishing Co., McCutcheon's, Detergents & Emulsifiers, (North American edition 1979); Schwartz, et al., Surface Active Agents, Their Chemistry and Technology, New York. Inter- science Publishers, 1949; U.S. Patent 3, 155,591 , Hilfer issued November 3, 1964; U. S. Patent 3,929,678, Laughlin et al., issued December 30, 1975; U S. Patent 3,959,461 , Bai- ley et al., issued May 25, 1976; and U. S. Patent 4,387,090, Bolich, Jr., issued June 7, 1983.

Among the quaternary ammonium-containing cationic surfactant materials useful herein are those of the general formula (I) 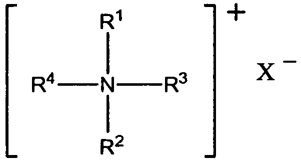 wherein R₁ to R₄ are independently an aliphatic group of from about 1 to about 22 carbon atoms or an aromatic, alkoxy, polyoxyalkylene, alkylamido, hydroxyalkyl, aryl or alkylaryl group having from about 1 to about 22 carbon atoms; and X⁻ is a salt-forming anion such as those selected from halogen, (e. g. chloride, bromide, iodide), acetate, citrate, lactate, glycolate, phosphate nitrate, sulfate, and alkylsulfate radicals.

The aliphatic groups may contain, in addition to carbon and hydrogen atoms, linkages or other groups, such as amino groups, or both. The longer chain aliphatic groups, e. g. those of about 12 carbons, or higher, can be saturated or unsaturated. Especially preferred are di-long chain (e.g., di-C₁₂-C₂₂, preferably C₁₆-C₁₈, aliphatic, preferably alkyl), di-short chain (e.g. C₁ - C₃ alkyl, preferably C₁ - C₂ alkyl) ammonium salts. Salts of primary, secondary and tertiary fatty amines are also suitable cationic surfactant materials. The alkyl groups of such amines preferably have from about 12 to about 22 carbon atoms, and may be substituted or unsubstituted. Such amines, useful herein, include stearamido propyl dimethyl amine, diethyl amino ethyl stearamide, dimethyl stearamine, dimethyl soyamine, soyamine, myristyl amine, tridecyl amine, ethyl stearylamine, N-tallowpropane diamine, ethoxylated (5 moles E.O.) stearylamine, dihydroxy ethyl stearylamine, and arachidyl behenylamine. Suitable amine salts include the halogen, acetate, phosphate, nitrate, citrate, lactate, and alkyl sulfate salts. Such salts include stearylamine hydrochloride, soyamine chloride, stearylamine formate, N-tallowpropane diamine dichloride and stearamidopropyl dimethylamine citrate. Cationic amine surfactants included among those useful in the present invention are disclosed in U.S. Patent 4,275,055, Nachtigal, et al., issued June 23, 1981. Preferred cationic surfactants are Genamin^{®} CTAC, i. e., cetyl trimethyl ammoniumchloride, esterquats as for example tetradecyl betainester chloride, diesterquats as for example dipalmitylethyl dimethylammoniumchloride (Armocare VGH70 of Akzo, Germany), or a mixture of distearoylethyl hydroxyethylmonium methosulfate and Cetearyl Alkohol (Dehyquart F-75 of Henkel, Germany).

Cationic surfactants (A) are preferably contained at levels of from about 0.1 % to about 3%, more preferably from about 0.2% to about 1.5%, most preferably from about 0.4% to about 0.8%, by weight of the composition.

### FATTY ALCOHOL

The compositions of the present invention comprise at least one fatty alcohol, preferably at least a nonvolatile low melting point fatty alcohol (B). Suitable fatty alcohols have a melting point of 30°C or less, preferably about 25°C or less, more preferably about 22°C or less. Preferred fatty alcohols are also nonvolatile. Fatty alcohols falling under the term "nonvolatile" have a boiling point at 1.0 atmospheres of at least about 260°C, preferably at least about 275°C, more preferably at least about 300°C. Suitable fatty alcohols include unsaturated monohydric straight chain fatty alcohols, saturated branched chain fatty alcohols, saturated C₈-C₁₂ straight chain fatty alcohols, and mixtures thereof. The unsaturated straight chain fatty alcohols will typically have one degree of unsaturation. Di- and tri- unsaturated alkenyl chains may be present at low levels, preferably at less than about 5% by total weight of the unsaturated straight chain fatty alcohol more preferably at less than about 2%, most preferably at less than about 1% by total weight of the unsaturated straight chain fatty alcohol. Preferably, the unsaturated straight chain fatty alcohols will have an aliphatic chain size of from C₁₂-C₂₂, more preferably from C₁₂-C₁₈, most preferably from C₁₆-C₁₈. Exemplary alcohols of this type include oleyl alcohol, and palmitoleic alcohol. The branched chain alcohols will typically have aliphatic chain sizes of from C₁₂-C₂₂, preferably C₁₄-C₂₀, more preferably C₁₆-C₁₈.

Exemplary branched chain alcohols for use herein include isostearyl alcohol, octyl dodeca- nol, and octyl decanol.

Examples of saturated C₈-C₁₂ straight chain alcohols include octyl alcohol, caprylic alcohol, decyl alcohol, and lauryl alcohol, low melting point fatty alcohols are preferably used at a level of from about 0.1 % to about 10%, by weight of the composition, more preferably from about 0.2% to about 5%, most preferably from about 0.5% to about 3%.

The present compositions are preferably limited to levels of monohydric saturated straight chain fatty alcohols, such as cetyl alcohol and stearyl alcohol, and other waxy fatty alcohols having melting points above 45°C, of no more than about 5% by weight of the composition, preferably no more than about 4% since the presence of such waxy fatty alcohols can adversely affect the shine benefits of the compositions according to the invention.

However, it may be desirable to use waxy fatty alcohols for their conditioning benefits. In the event that such saturated fatty alcohols are present, the weight ratio of the liquid to waxy fatty alcohols is preferably no greater than about 0.25, more preferably no greater than about 0.15, more preferably than about 0.10.

It can be especially preferred, if the cosmetic composition comprises at least one fatty alcohol having a melting point of more than 30°C and optionally at least one fatty alcohol having a melting point of 30°C or less, wherein the weight ratio of fatty alcohols having melting points to fatty alcohols having melting points of 30°C or lower is 0.25 or less in the case that the composition comprises at least one fatty alcohol having a melting point of more than 30°C and at least one fatty alcohol having a melting point of 30°C or less.

The total amount of fatty alcohols in the composition is preferably about 0.5 to about 5.0 % by weight, more preferably from about 1.0 to about 4.0% by weight, and most preferably from about 1.5 to about 3.0% by weight.

### CARBON DIOXIDE

Carbon dioxide is present as a propellant in the inner bag of the container as a part of the cosmetic composition. It is possible according to the invention that carbon dioxide is not the sole propellant, it can be in admixture with any other propellant, e.g., propane, butane, isobutane, dimethyl ether or N₂O, or mixtures of two or more of the above, as long as such a mixture does not show properties which are adverse to the object of the invention.

It can be preferred that carbon dioxide is the sole propellant, however, in this preferred case it is also possible that small amounts of up to about 1 % by weight of other propellants, such as propane, butane, isobutane, dimethyl ether or N₂O, may be present.

The amount of carbon dioxide in the cosmetic composition is preferably 0.5 to 5.0 % by weight, more preferably 1.0 to 3.0 % by weight and most preferably 1 .5 to 2.5 % by weight of the composition.

### WATER PHASE

The cosmetic composition forming a part of the invention can contain water. In this case, the water phase preferably contains about 70 to about 98% by weight, more preferably from about 85 to about 96% by weight, and most preferably from about 90 to about 95% by weight of water.

The water phase can optionally include other liquid, water-miscible or water-soluble solvents such as lower alkyl alcohols, e. g. C₁ -C₅ alkyl monohydric alcohols, preferably C₂- C₃ alkyl alcohols. However, the liquid fatty alcohol must be miscible in the aqueous phase of the composition. Said fatty alcohol can be miscible in the aqueous phase or can be made miscible through the use of co-solvents or surfactants.

The cosmetic composition preferably has a viscosity at 25°C of at least about 50 mPas, preferably from about 100 mPas to about 1,500 mPas, more preferably from about 200 mPas to about 1,000 mPas. Viscosity is determined by HAAKE Rotation Viscometer VT 550 with cooling/heating vessel and sensor systems according to DIN 53019 (MV-DIN), shear rate is 12.9 s⁻¹.

The compositions of the present invention preferably have a pH of from about 2.5 to about 11, more preferably from about 3 to about 9.5, most preferably from about 4.0 to about 7.0.

### CATIONIC POLYMER CONDITIONING AGENT

The compositions of the present invention can also contain one or more cationic polymer conditioning agents. The cationic polymer conditioning agent will preferably be water soluble. Cationic polymers are typically used in the same ranges as disclosed above for cationic surfactants.

By "water soluble" cationic organic polymer, what is meant is a polymer which is sufficiently soluble in water to form a substantially clear solution to the naked eye at a concentration of 0.1 % in water (distilled or equivalent) at 25°C. Preferably, the polymer will be sufficiently soluble to form a substantially clear solution at 0.5% concentration, more preferably at 1 .0% concentration.

As used herein, the term "polymer" shall include materials whether made by polymerization of one type of monomer or made by two (i.e., copolymers) or more types of monomers.

The cationic polymers hereof will generally have a weight average molecular weight which is at least about 5,000, typically at least about 10,000, and is less than about 10 million. Preferably, the molecular weight is from about 100,000 to about 2 million. The cationic polymers will generally have cationic nitrogen-containing moieties such as quaternary ammonium or cationic amino moieties, or a mixture thereof.

The cationic charge density is preferably at least about 0.1 meq/gram, more preferably at least about 1.5 meq/gram, even more preferably at least abut 1.1 meq/gram, most preferably at least about 1.2 meq/gram. Cationic charge density of the cationic polymer can be determined according to the Neldahl Method. Those skilled in the art will recognize that the charge density of amino-containing polymers may vary depending upon pH and the isoelectric point of the amino groups. The charge density should be within the above limits at the pH of intended use. Any anionic counterions can be utilized for the cationic polymers so long as the water solubility criteria is met. Suitable counterions include halides (e. g., Cl, Br, I, or F, preferably Cl, Br, or I), sulfate, and methylsulfate. Others can also be used, as this list is not exclusive. The cationic nitrogen-containing moiety will be present generally as a substituent, on a fraction of the total monomer units of the cationic hair conditioning polymers. Thus, the cationic polymer can comprise copolymers, terpolymers etc. of quaternary ammonium or cationic amine-substituted monomer units and other non-cationic units referred to herein as spacer monomer units.

Suitable cationic polymers include, for example, copolymers of vinyl monomers having cationic amine or quaternary ammonium functionalities with water soluble spacer monomers such as acrylamide, methacrylamide, alkyl and dialkyl acrylamides, alkyl and dialkyl methacrylamides, alkyl acrylate, alkyl methacrylate, vinyl caprolactone, and vinyl pyrrolidone. The alkyl and dialkyl substituted monomers preferably have C1-C7 alkyl groups, more preferably C1 -C3 alkyl groups.

Other suitable spacer monomers include vinyl esters, vinyl, alcohol (made by hydrolysis of polyvinyl acetate), maleic anhydride, propylene glycol, and ethylene glycol.

The cationic amines can be primary, secondary, or tertiary amines, depending upon the particular species and the pH of the composition. In general, secondary and tertiary amines, especially tertiary, amines, are preferred. Amine-substituted vinyl monomers can be polymerized in the amine form, and then optionally can be converted to ammonium by a quaternization reaction. Amines can also be similarly quaternized subsequent to formation of the polymer. For example, tertiary amine functionalities can be quaternized by reaction with a salt of the formula R'X wherein R' is a short chain alkyl, preferably a C₁ -C₇ alkyl, more preferably a C₁-C₃ alkyl, and X is an anion which forms a water soluble salt with the quaternized ammonium.

Suitable cationic amino and quaternary ammonium monomers include, for example, vinyl compounds substituted with dialkylaminoalkyl acrylate, dialkylaminoalkyl methacrylate, monoalkylaminoalkyl acrylate, monoalkylaminoalkyl methacrylate, trialkyl methacryloxyal- kyl ammonium salt, trialkyl acryloxyalkyl ammonium salt, diallyl quaternary ammonium salts, and vinyl quaternary ammonium monomers having cyclic cationic nitrogen- containing rings such as pyridinium, imidazolium, and quaternized pyrrolidone, e.g. alkyl vinyl imidazolium, alkyl vinyl pyridinium, alkyl vinyl pyrrolidone salts. The alkyl portions of these monomers are preferably lower alkyls such as the C₁-C₃ alkyls, more preferably C₁ and C₂ alkyls. Suitable amine-substituted vinyl monomers for use herein include dialkylaminoalkyl acrylate, dialkylaminoalkyl methacrylate, dialkylaminoalkyl acrylamide, and dialkylaminoalkyl methacrylamide, wherein the alkyl groups are preferably C₁-C₇ hydrocarbyls, more preferably C₁-C₃ alkyls.

The cationic polymers hereof can comprise mixtures of monomer units derived from amine- and/or quaternary ammonium-substituted monomer and/or compatible spacer monomers.

Suitable cationic hair conditioning polymers include, for example: copolymers of 1 -vinyl-2-pyrrolidone and 1-vinyl-3-methylimidazolium salt (e.g., chloride salt) (referred to in the industry by the Cosmetic, Toiletry, and Fragrance Association, "CTFA", as Polyquaternium-16), such as those commercially available from BASF Wyandotte Corp. (Parsippany, NJ, USA) under the LUVIQUAT tradename (e. g. LUVIQUAT FC 370); copolymers of 1-vinyl-2-pyrrolidone and dimethylaminoethyl methacrylate (referred to in the industry by CTFA as Polyquaternium-1 1) such as those commercially available from Gaf Corporation (Wayne, NJ, USA) under the GAFQUAT tradename (e.g., GA17QUAT 755N); cationic diallyl quaternary ammonium-containing polymers, including, for example, dimethyl diallyl ammonium chloride homopolymer and copolymers of acrylamide and dimethyl diallyl ammonium chloride, referred to in the industry (CTFA) as Polyquaternium-6 and Polyquaternium-7, respectively; and mineral acid salts of amino-alkyl esters of homo- and co-polymers of unsaturated carboxylic acids having from 3 to 5 carbon atoms, as described in U.S. Patent 4,009,256, incorporated herein by reference. Other cationic polymers that can be used include polysaccharide polymers, such as cationic cellulose derivatives and cationic starch derivatives. Cationic polysaccharide polymer materials suitable for use herein include those of the formula: 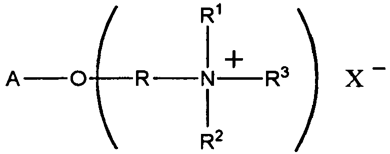 wherein: A is an anhydroglucose residual group, such as a starch or cellulose anhydroglucose residual, R is an alkyene, oxyalkylene, polyoxyalkylene or hydroxyalkylene group, or combination thereof, R1, R2, and R3 independently are alkyl, aryl, alkylaryl, arylalkyl, alkoxyalkyl, or alkoxyaryl groups, each group containing up to about 18 carbon atoms, and the total number of carbon atoms for each cationic moiety (i.e., the sum of carbon atoms in R1, R2 and R3) preferably being about 20 or less, and X is an anionic counterion, as previously described.

Cationic cellulose is available from Amerchol Corp. (Edison, NJ, USA) in their Polymer JR^{©} and LR^{©} series of polymers, as salts of hydroxyethyl cellulose reacted with trimethyl ammonium substituted epoxide, referred to in the industry (CTFA) as Polyquaternium-10.

Another type of cationic cellulose includes the polymeric quaternary ammonium salts of hydroxyethyl cellulose reacted with lauryl dimethyl ammonium-substituted epoxide, referred to in the industry (CTFA) as Polyquaternium-24. These materials are available from Amerchol Corp. (Edison, NY, USA) under the tradename Polymer LM-200^{©}.

Other cationic polymers that can be used include cationic guar gum derivatives, such as guar hydroxypropyltrimonium chloride (commercially available from Celanese Corp. in their JaguarR^{©} series).

Other materials include quaternary nitrogen-containing cellulose ethers (e. g. as described in U.S. Patent 3,962,418, incorporated by reference herein), and copolymers of etherified cellulose and starch (e. g. as described in U.S. Patent 3,958,581, incorporated by reference herein).

As discussed above, the cationic polymer thereof is water soluble. This does not mean, however, that it must be soluble in the composition. Preferably the cationic polymer can be either soluble in the composition, or in a complex coacervate phase in the composition formed by the cationic polymer and anionic material. Complex coacervates of the cationic polymer can be formed with anionic surfactants or with anionic polymers that can optionally be added to the compositions hereof (e. g. sodium polystyrene sulfonate).

### SILICONE CONDITIONING AGENTS

The compositions thereof can also include nonvolatile soluble or insoluble silicone conditioning agents. By soluble what is meant is that the silicone conditioning agent is miscible with the aqueous carrier of the composition so as to form part of the same phase. By insoluble what is meant is that the silicone from a separate, discontinuous phase from the aqueous carrier, such as in the form of an emulsion or a suspension of droplets of the silicone.

Soluble silicones include silicone copolyols, such as dimethicone copolyols, e.g. polyether siloxane modified polymers, such as polypropylene oxide, polyethylene oxide modified polydimethylsiloxane, wherein the level of ethylene and/or propylene oxide sufficient to allow solubility in the composition.

Preferred, however, are insoluble silicones. The insoluble silicone hair conditioning agent for use herein will preferably have viscosity of from about 1 ,000 to about 2,000,000 centistokes at 25°C, more preferably from about 10,000 to about 1,800,000, even more preferably from about 100,000 to about 1,500,000 centistokes at 25°C. The viscosity can be measured by means of a glass capillary viscometer as set forth in Dow Coming Corporate Test Method CTM0004, July 20, 1970.

Suitable insoluble, nonvolatile silicone fluids include polyalkyl siloxanes, polyaryl siloxanes, polyalkylaryl siloxanes, polyether siloxane copolymers, and mixtures thereof. Other insolu- ble, nonvolatile silicone fluids having hair conditioning properties can also be used. The term "nonvolatile" as used herein shall mean that the silicone has a boiling point of at least about 260⁹C, preferably at least about 275°C, more preferably at least about 300°C. Such materials exhibit very low or no significant vapor pressure at ambient conditions. The term "silicone fluid" shall mean flowable silicone materials having a viscosity of less than 1,000,000 centistokes at 25°C. Generally, the viscosity of the fluid will be between about 5 and 1,000,000 centistokes at 250, preferably between about 10 and about 300,000.

The preferred silicones are polydimethyl siloxane, polydiethylsiloxane, and polymethyl-phenylsiloxane. Polydimethylsiloxane is especially preferred. The nonvolatile polyalkylsiloxane fluids that may be used include, for example, polydimethylsiloxanes. These siloxanes are available, for example, from the General Electric Company in their ViscasilR and SF 96 series, and from Dow Coming in their Dow Coming 200^{®} series.

The polyalkylaryl siloxane fluids that may be used, also include, for example, polymethyl-phenylsiloxanes. These siloxanes are available, for example, from the General Electric Company as SF 1075 methyl phenyl fluid or from Dow Coming as 556 Cosmetic Grade Fluid or diquaternary silicones as for example INCI Quaternium-80 (e. g. Abil^{®} Quat 3272 or Abil^{®} Quat 3270 of Th. Goldschmidt AG, Germany).

Especially preferred, for enhancing the shine characteristics of hair, are highly arylated silicones, such as highly phenylated polyethyl silicone having refractive indices of about 1 .46 or higher, especially about 1 .52 or higher. When these high refractive index silicones are used, they should be mixed with a spreading agent, such as a surfactant or a silicone resin, as described below to decrease the surface tension and enhance the film forming ability of the material.

The polyether siloxane copolymers that may be used include, for example, a polypropylene oxide modified polydimethylsiloxane (e.g., Dow Corning Doc-1248^{®}) although ethylene oxide or mixtures of ethylene oxide and propylene oxide may also be used. The ethylene oxide and polypropylene oxide level should be sufficiently low to prevent solubility in the composition hereof.

Another silicone hair conditioning material that can be especially useful in the silicone conditioning agents is insoluble silicone gum. The term "silicone gum", as used herein, means polyorganosiloxane materials having a viscosity at 25°C of greater than or equal to 1,000,000 centistokes. Silicone gums are described by Petrarch and others including U.S. Patent 4,152,416, Spitzer et al., issued May 1, 1979 and Noll, Walter, Chemistry and Technology of Silicones, New York: Academic Press 1968. Also describing silicone gums are General Electric Silicone Rubber Product Data Sheets SE 30, SE 33, SE 54 and SE 76. All of these described references are incorporated herein by reference. The "silicone gums" will typically have a mass molecular weight in excess of about 200,000, generally between about 200,000 and about 1,000,000. Specific examples include polydimethylsiloxane, (polydimethylsiloxane) (methylvinylsiloxane) copolymer, poly(dimethylsiloxane) (diphenyl siloxane) (methylvinylsiloxane) copolymer and mixtures thereof.

Preferably the silicone hair conditioning agent comprises a mixture of a polydimethylsiloxane gum, having a viscosity greater than about 1,000,000 centistokes and polydimethylsiloxane fluid having a viscosity of from about 10 centistokes to about 100,000 centistokes at 25°C, wherein the ratio of gum to fluid is from about 30:70 to about 70:30, preferably from about 40:60 to about 60:40.

An optional ingredient that can be included in the silicone conditioning agent is silicone resin. Silicone resins are highly crosslinked polymeric siloxane systems. The crosslinking is introduced through the incorporation of trifunctional and tetrafunctional silanes with mono- functional or difunctional, or both, silanes during manufacture of the silicone resin. As is well understood in the art, the degree of crosslinking that is required in order to result in a silicone resin will vary according to the specific silane units incorporated into the silicone resin. In general, silicone materials which have a sufficient level of trifunctional and tetrafunctional siloxane monomer units (and hence, a sufficient level of crosslinking) such that they dry down to a rigid, or hard, film are considered to be silicone resins. The ratio of oxygen atoms to silicon atoms is indicative of the level of crosslinking in a particular silicone material. Silicone materials which have at least about 1.1 oxygen atoms per silicon atom will generally be silicone resins herein.

Preferably, the ratio of oxygen : silicon atoms is at least about 1 .2 :1.0.

Silanes used in the manufacture of silicone resins include monomethyl-, dimethyl-, trimethyl-, monophenyl-, diphenyl-, methylphenyl-, monovinyl-, and methylvinylchlorosilanes, and tetrachlorosilane, with the methyl- substituted silanes being most commonly utilized. Preferred resins are offered by General Electric as GE SS4230 and SS4267^{®}. Commercially available silicone resins will generally be supplied in a dissolved form in a low viscosity volatile or nonvolatile silicone fluid. The silicone resins for use herein should be supplied and incorporated into the present compositions in such dissolved form, as will be readily apparent to those skilled in the art. Silicone resins can enhance deposition of silicone on the hair and can enhance the glossiness of hair with high refractive index volumes.

Silicone materials and silicone resins in particular, can conveniently be identified according to a shorthand nomenclature system well known to those skilled in the art as "MDTQ" nomenclature. Under this system, the silicone is described according to presence of various siloxane monomer units which make up the silicone. Briefly, the symbol M denotes the monofunctional unit (CH3)₃SiO_{.5}; D denotes the difunctional unit (CH3)₂SiO; T denotes the trifunctional unit (CH3)SiO_{1.5}; and Q denotes the quadri- or tetra-functional unit SiO₂. Primes of the unit symbols, e.g., M', D', 'T, and Q' denote substituents other than methyl, and must be specifically defined for each occurrence. Typical alternate substituents in- elude groups such as vinyl, phenyls, amines, hydroxyls, etc. The molar ratios of the various units, either in terms of subscripts to the symbols indicating the total number of each type of unit in the silicone (or an average thereof) or as specifically indicated ratios in combination with molecular weight complete the description of the silicone material under the MDTQ system. Higher relative molar amounts of T, Q, T' and/or Q' to D, D', M and/or or M' in a silicone resin is indicative of higher levels of crosslinking. As discussed before, however, the overall level of crosslinking can also be indicated by the oxygen to silicon ratio.

The silicone resins for use herein which are preferred are MQ, MT, MTQ, MQ and MDTQ resins. Thus, the preferred silicone substituent is methyl. Especially preferred are MQ resins wherein the M:Q ratio is from about 0.5 : 1.0 to about 1.5 : 1.0 and the average molecular weight of the resin is from about 1,000 to about 10,000.

The silicone hair conditioning agent can be used in the compositions hereof at levels of from about 0.1 % to about 5% by weight of the composition, preferably from about 0.3 % to about 3%, more preferably from about 0.5% to about 3.0%, most preferably from about 1 .0% to about 3.0 % by weight.

### ADDITIONAL CONDITIONING AGENTS

The compositions of the present invention can also comprise one or more additional conditioning agents, such as those selected from the group consisting of avocado oil, fatty acids, isopropyl myristate, lanolin, apple wax, bees wax or jojoba oil, phospholipides, e. g. lecithines or ceramides; vaseline nonvolatile hydrocarbons and hydrocarbon esters. Useful are also imidazolidinyl derivatives as for example INCI Quaternium-87 (Rewoquat^{®} W 575 of Witco, Germany).

The components hereof can comprise from 0.1 % to about 20%, preferably, from about 0.1 % to about 10%, more preferably from about 0.5% to about 5%, of additional conditioning agents.

### OTHER INGREDIENTS

The compositions herein can contain a variety of other optional components suitable for rendering such compositions more cosmetically or aesthetically acceptable or to provide them with additional usage benefits. Such conventional optional ingredients are well-known to those skilled in the art.

A wide variety of additional ingredients can be formulated into the present composition. These include: other conditioning agents, e. g. betaine, carnitin esters, creatine, amino acids, peptides, proteines and vitamines; hair-hold polymers, detersive surfactants such as anionic, nonionic, amphoteric, and zwitterionic surfactants; thickening agents and suspending agents, such as xanthan gum, guar gum, hydroxyethyl cellulose, methyl cellulose, hydroxyethylcellulose, starch and starch derivatives, viscosity modifiers such as methanolamides of long chain fatty acids, cocomonoethanol amide, salts such as sodium potassium chloride and sulfate and crystalline suspending agents, and pearlescent aids such as ethylene glycol distearate; UV-filters such as p-methoxy cinnamic acid isoamylester, lipophilc cinnamic acid esters, salicylic acid esters, 4-amino benzoic acid derivatives or hydrophilic sulfonic acid derivatives of benzophenones or 3-benzyliden campher; antioxidants such as tocopheroles; preservatives such as benzyl alcohol, methyl paraben, propyl paraben and imidazolidinyl urea; polyvinyl alcohol; ethyl alcohol; pH adjusting agents, such as citric acid, formic acid, glyoxylic acid, acetic acid, lactic acid, pyruvic acid, sodium citrate, succinic acid, phosphoric acid, sodium hydroxide, sodium carbonate; salts, in general, such as potassium acetate and sodium chloride; coloring agents, such as any of the FD&C or D&C dyes; hair oxidizing (bleaching) agents, such as hydrogen peroxide, perborate and persulfate salts; hair reducing agents, such as the thioglycolates; perfumes, sequestering agents, such as disodium ethylenediamine tetra-acetate, and polymer plasticizing agents, such as glycerin, disobutyl adipate, butyl stearate, and propylene glycol.

Such optional ingredients generally are used individually at levels from about 0.01% to about 10.0%, preferably from about 0.05% to about 5.0% of the composition.

The compositions of the present invention can further comprise from about 0.1 % to about 2%, more preferably from about 0.2% to about 1%, and most preferably from about 0.5% to about 1% of a polymer thickening agent. They can still provide a good perception of spreading upon application to the hair.

### METHOD OF USE

The cosmetic compositions of the present invention are used in conventional ways to provide beneficial effects to the hair, especially conditioning and shine benefits. The method of use depends upon the type of aerosol cream composition employed, but generally involves application of an effective amount of the product to the hair, which may then be rinsed from the hair (as in the case of hair rinses) or allowed to remain on the hair (as in the case of leave- in products). By "effective amount" it is meant an amount sufficient enough to provide a hair conditioning and/or hair shine benefit. In general, from about 1g to about 50g is applied to the hair on the scalp. The composition is distributed throughout the hair by, typically by rubbing or massaging the hair and scalp with ones' hands or by another's hands.

Preferably, the cosmetic composition is applied to wet or damp hair prior to drying of the hair. After such compositions are applied to the hair, the hair is dried and styled in accordance with the desires of the user and in the usual ways of the user. Alternately, the composition is applied to dry hair, and the hair is then combed or styled in accordance with the desires of the user.

The cosmetic composition according to the present invention can be used for leave-in and rinse-off applications as well. In the latter case, the period of action of the composition depends on the temperature (about 20°C to 50°C) and is 1 minute to 60 minutes and preferably 5 minutes to 20 minutes. The composition can also be used as a pre-treating agent before dyeing or before a permanent wave treatment.

The benefits of the invention result from the unique combination of a container comprising an inner bag and an outer container, which encloses the inner bag, while the inner bag has a valve mechanism attached which is movable between an open position and a closed position.

The container 10 in which the cosmetic composition is contained comprises an inner bag 12 filled with the cosmetic composition as specified above, an outer container 14 disposed on the outside of the inner bag 12 and enclosing the inner bag, and a valve mechanism 16 sealing the container 14 and the inner bag 12. The outer container 14 is formed from metal or plastic or the like, and a propellant such as air, nitrogen, carbon dioxide, or an organic propellant such as dimethylether, ethane, propane, butane or the like or any other suitable compressed gas is filled contained in a space between the outer container 14 and the inner bag 12. The pressure of the propellant is preferably set to 0.3 to 1.0 MPa, preferably to about 0.8 MPa, from the perspective of stably discharging the content of the bag until the preferably complete exhaustion of the composition contained in the bag.

The inner bag 12 is preferably flexible, and can be made from a single material or from a composite material, which preferably comprises at least a polymeric layer and a layer which acts as a gas barrier, e.g., made from metal, such as Aluminum. Preferably the inner material of the bag is inert to the contents of the composition, and more preferably the inner material is also impenetrable by the contents of the composition in the bag. It is further preferred if the inner bag comprises a layer of a material which is essentially impermeable to the propellant inside of the bag. It is also preferred if the inner bag comprises a layer of a material which is essentially impermeable to the propellant outside of the bag which generally is not intended to be mixed with the composition in the inner bag during storage. Mixing of the propellant in the bag and the propellant outside of the bag can be inappropriate to the properties of the cosmetic composition in the bag, e.g. due to the fact that the propellant outside of the bag influences the properties of the cosmetic composition during storage or after discharge from the bag in a negative way, or it can be inappropriate due to the fact that the propellant outside of the bag and inside of the bag are not compatible, e.g., due to a chemical reaction between the propellants.

This does, however, not preclude the possibility that the propellant inside of the bag and the propellant outside of the bag are mixed upon dispensing of the cosmetic composition when the dispensing valve is triggered to open and to dispense the contents of the inner bag. A mixing channel or another appropriate measure can be used in such a case to mix the propellant in the container outside of the inner bag with the dispensed cosmetic composition comprising carbon dioxide, if this is desired.

The inner bag 12 can comprise flat lateral edges 18 and might additionally also comprise a bottom fold 20 directed towards the upper end of the bag 12 in order to allow a controlled collapse of the bag. In the area of the bottom fold 20, the inner bag 12 can comprise two flat triangular portions 22 each extending from the bottom edge 24 to the lateral edge 18 with an angle of about 45 °. This can further facilitate the collapse of the bag 12, when compressed by the pressure of the outside propellant.

Generally, all types of valve systems can be part of the inner bag. In an illustrative embodiment the valve mechanism 16 comprises a housing 26, a valve stem 28, a spring 30, a valve plate 32, an inner sealing 34 and an outer sealing 36. The valve stem 28 comprises at least on lateral opening 38 and is moveable up and down within the housing 26. The spring 30 is disposed between the lower end portion 40 of the valve stem 28 and the housing 26 and biases the valve stem 28 in an upward direction towards the valve plate 32 which is disposed at the upper end of the housing 26. The valve plate 32 comprises two recesses 42, 44 extending in a circumferential direction of the valve plate 32 and arranged coaxially. An axial opening 46 is located in the central portion of the inner recess 42. The inner sealing 34 is disposed within the inner recess 42 and is attached to the valve plate 32. The inner sealing 34 is adapted to engage the valve stem 28 such that the lateral opening 38 of the valve stem 28 is covered and blocked, respectively. The outer sealing 36 is disposed in the second or outer recess 44 of the valve plate 32. The valve stem 28 comprises a passage 48 in the central axial portion thereof connected to the lateral opening 38 on the one side and connectable to a corresponding passage of a dispenser cap on the other side. Particularly, the spring 30 causes the valve stem 28 to be pressed into a position, wherein the inner sealing 34 blocks the lateral opening 38 from the interior space of the housing 26 of the valve mechanism 16. This means, a flow path from the interior space of the housing 26 along the valve stem 28 and through the lateral opening 38 of the valve stem 28 is blocked by the inner sealing 34. The valve mechanism 16 is fixed to the inner bag 12 at an upper end thereof such that a lower end of the housing 26 of the valve mechanism 16 is gas tight covered by the upper edge of the inner bag 12.

Further, the inner bag 12 and the valve mechanism 16 are attached to the outer container 14 such that an upper end of the outer container 14 engages the outer sealing 44 of the valve plate 32 in a gas tight manner. Accordingly, the interior of the inner bag 12 and the space between the outer container 14 and the inner bag 12 are each independently sealed. A dispenser cap, which is not shown, having an actuator is attached to the valve plate 32 such that the actuator engages the valve stem 28. For this purpose, the actuator is inserted into the central opening 46 of the valve plate 32. The actuator comprises a passage extending from a jetting opening of the dispenser cap to a lower end thereof. In an alternative embodiment, the valve mechanism may comprise a valve seat and a valve element instead of the valve stem.

The container 10 can operate as follows. First, with the dispenser cap not pressed, the cosmetic composition filled into the interior of the inner bag 12 and the compressed gas filled into the space between the outer container 14 and the inner bag 12 are each sealed and stored because the actuator only contacts the valve stem 28 but does not press it away out of the contact with the inner sealing 34. Consequently, the valve 16 is in a closed position and a flow path through the valve housing 26, the lateral opening 38 of the valve stem 28, and the passage 48 within the valve stem 28 is blocked.

When the dispenser cap and the actuator are pressed down, the valve stem 28 is displaced downward against the biasing force of the spring 30 and the inner sealing 34 remains stationary. Thus, the valve stem 28 moves within the inner sealing 34 while contacting the same. The movement continues until the lateral opening 38 of the valve stem 28 is uncovered by the inner sealing 34 and a flow path between the valve housing 26 and the valve stem 28 through the lateral opening 38 is opened. Thus, the interior of the inner bag 12 and the flow path inside the valve housing 26 become linked such that the hair composition filled into the inner bag 12 passes through the flow path and is jetted out of the dispenser cap by the pressure of the compressed gas. At the same time, the inner bag 12 is compressed. Due to the flat lateral edges 18, the bottom fold 20 and the flat portions 24 at the bottom edge, the inner bag 12 is collapsed in a controlled manner and takes the form of a flat sheet if totally compressed.

If the downward pressure on the dispenser cap is removed, the spring 30 causes the valve stem 28 to return to the closed position, wherein the inner sealing 34 blocks the lateral opening 38 of the valve stem 28.

### COMPARISON EXPERIMENTS

### Example of the invention:

**Cosmetic composition:**

| % by weight | |
|---|---|
| 0,3 | dexpanthenol |
| 93,8 | water purified |
| 0,4 | cetrimonium chloride |
| 0,1 | salt composition |
| 0,3 | preservative |
| 0,1 | gellant |
| 1,8 | cetearyl alcohol |
| 0,5 | petrolatum |
| 0,4 | mineral oil |
| 0,3 | perfume |
| 2,0 | carbon dioxide |

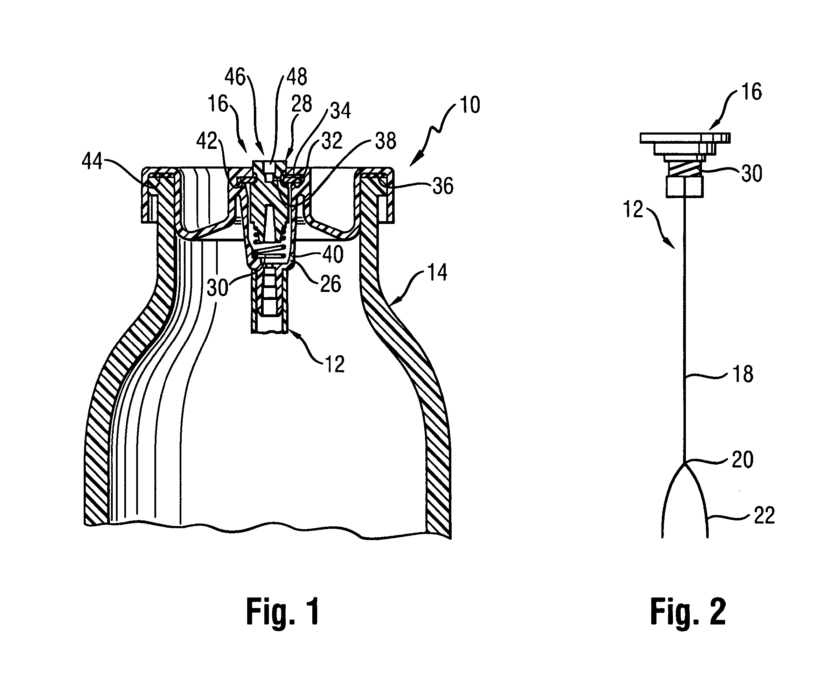

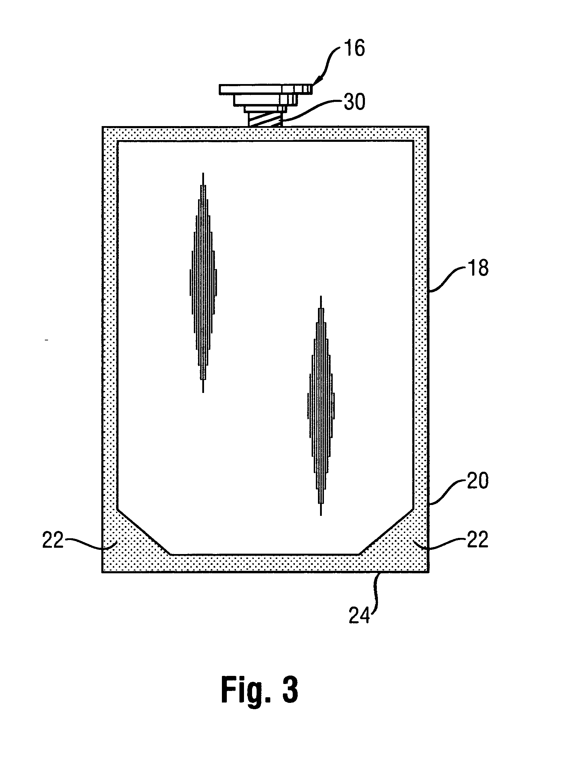

Container:
Compare Fig. 1-3;
200 ml Al can (#98938662); Coster spout (#95936671.001);
Seaquist Valve, ref. to IPMS 99401377; Tinplate mounting cup stem 4,02 x 3,8 Fast fill System
Seaquist Valve-Pouch (bag) size D6, 150 ml;
4-layer bag, Foil/Lam: PE-64 (inner layer), adhesive, aluminum-layer, PET-layer (outer layer)

### Comparative example:

**Cosmetic composition:**

| % by weight | |
|---|---|
| 0,3 | dexpanthenol |
| 93,3 | water purified |
| 0,4 | cetrimonium chloride |
| 0,1 | salt composition |
| 0,3 | preservative |
| 0,1 | gellant |
| 1,8 | cetearyl alcohol |
| 0,5 | petrolatum |
| 0,4 | mineral oil |
| 0,3 | perfume |
| 2,5 | carbon dioxide |

Container:
Seaquist 200 ml Al can, without inner bag

### Exhaustion of the cosmetic composition

The cosmetic composition provided in a container according to the invention (example of the invention) and the cosmetic composition according to WO 2007/010487 provided in a regular aerosol can (comparative example) were compared with respect to their exhaustion properties.

The weights/amounts of cosmetic composition residuals remaining in the respective containers were determined by using a calibrated laboratory balance with a resolution to 0,01 grams. Prior to testing all samples were stored for a minimum of 24 hours at 22° C +/- 3°C and 65% RH +/-10% RH.

Table 1 refers to the cosmetic composition according to the comparative example and shows the amounts/weights of residuals of cosmetic composition remaining in the container after the propellant was exhausted.

**Table 1**

| Sample No. | remaining weight [g] |
|---|---|
| 1 | 30 |
| 2 | 18 |
| 3 | 43 |
| 4 | 23 |
| 5 | 29 |
| 6 | 55 |
| 7 | 36 |
| 8 | 32 |
| Average | **33,25** |
| STD | 11,61 |

Tables 2 and 3 show the amounts/weights of cosmetic composition according to the example of the invention remaining in the container after one, two, three, six and twelve months of storing and discharging the composition at 25° C and 40° C, respectively, until the propellant was exhausted.

**Table 2**

| **25° C** | | Initial | 1 month | 2 months | 3 months | 6 months | 12 months |
|---|---|---|---|---|---|---|---|
| Sample No. | empty pack [g] | empty pack [g] | weight empty pack [g] | weight empty pack [g] | weight empty pack [g] | weight empty pack [g] | weight empty pack [g] |
| 1 | 48,30 | 49,60 | 48,00 | 48,09 | 49,20 | 48,73 | 48,83 |
| 2 | 48,30 | 49,52 | 48,99 | 48,32 | 48,15 | 48,04 | 48,91 |
| 3 | 48,30 | 48,95 | 49,39 | 49,48 | 48,95 | 49,27 | 49,00 |
| 4 | 48,30 | 49,69 | 49,53 | 49,60 | 48,93 | 48,93 | 48,84 |
| 5 | 48,30 | 49,81 | 49,32 | 49,30 | 49,80 | 48,47 | 49,42 |
| 6 | 48,30 | 49,12 | 50,30 | 49,30 | 49,42 | 49,42 | 49,00 |
| 7 | 48,30 | 48,87 | 48,92 | 48,99 | 49,67 | 48,97 | 48,90 |
| 8 | 48,30 | 48,90 | 49,41 | 48,72 | 48,87 | 48,53 | 49,46 |
| 9 | 48,30 | 48,60 | 48,94 | 48,92 | 48,97 | 48,98 | 49,91 |
| 10 | 48,30 | 50,25 | 49,30 | 48,64 | 50,20 | 48,84 | 49,07 |
| Average | 48,30 | 49,33 | 49,21 | 48,94 | 49,22 | 48,82 | 49,13 |
| STD | 0,00 | 0,52 | 0,58 | 0,50 | 0,58 | 0,40 | 0,35 |
| remaining weight [g] | | **1,03** | **0,91** | **0,64** | **0,92** | **0,52** | **0,83** |

**Table 3**

| **40° C** | | Initial | 1 month | 2 months | 3 months | 6 months | 12 months |
|---|---|---|---|---|---|---|---|
| Sample No. | empty pack [g] | empty pack [g] | weight empty pack [g] | weight empty pack [g] | weight empty pack [g] | weight empty pack [g] | weight empty pack [g] |
| 1 | 48,30 | 49,03 | 49,74 | 49,01 | 48,56 | 49,79 | 48,83 |
| 2 | 48,30 | 48,70 | 49,04 | 48,87 | 49,47 | 49,77 | 50,34 |
| 3 | 48,30 | 49,30 | 48,98 | 48,85 | 49,94 | 50,52 | 49,42 |
| 4 | 48,30 | 49,60 | 48,93 | 48,60 | 49,40 | 50,26 | 49,65 |
| 5 | 48,30 | 49,30 | 49,01 | 49,87 | 49,57 | 50,42 | 49,96 |
| 6 | 48,30 | 48,73 | 50,15 | 49,55 | 48,60 | 49,38 | 49,55 |
| 7 | 48,30 | 50,20 | 48,47 | 49,20 | 49,01 | 48,81 | 49,27 |
| 8 | 48,30 | 49,30 | 49,16 | 49,19 | 49,71 | 49,30 | 49,28 |
| 9 | 48,30 | 49,60 | 48,56 | 49,63 | 49,45 | 48,86 | 48,72 |
| 10 | 48,30 | 48,80 | 49,04 | 49,62 | 49,27 | 49,17 | 51,85 |
| Average | 48,30 | 49,26 | 49,11 | 49,24 | 49,30 | 49,63 | 49,69 |
| STD | 0,00 | 0,47 | 0,50 | 0,41 | 0,45 | 0,62 | 0,90 |
| remaining weight [g] | | **0,96** | **0,81** | **0,94** | **1,00** | **1,33** | **1,39** |

The results in tables 1 to 3 clearly show that the weight/amount of composition remaining in the container according to the invention is decreased in comparison to the comparative example. Thus, the present invention leads to an increased level of exhaustion, i.e. the exhaustible amount of the composition according to the example of the invention is significantly increased.

### Sensory Test

Further, sensory tests show performance advantages of the cosmetic composition according to the invention as compared to the state of the art (cf. comparative example). Sensory tests enable trained and experienced panelists to evaluate the effects of hair products in comparison with a defined standard. In the course of the test a sample of the tested product is applied to defined hair strands, and then directly compared to the respective untreated hair strand with respect to various technical hairstyling criteria. The test samples were applied to defined hair strands, thus enabling direct comparison under identical test conditions (identical hair structure, hair color etc.). Performance of the cosmetic composition according to the example of the invention was compared with performance of the cosmetic composition of the comparative example. Both compositions were applied as rinse-off foam-conditioners.

The sensory tests were carried out by 30 trained and experienced panelists for the criteria listed in table 4. The numbers in the table indicate how each criterion was judged on a scale between 0 and 10.

Foam extension was evaluated by applying tennis-ball-sized amounts of the respective cosmetic compositions on the palm of a hand and monitoring volume-expansion of the compositions directly after applying the foam onto the palm.

Foam compactness was evaluated by assessing the level of difficulty of placing the foam ball from one hand to the other.

Foam persistency was evaluated by monitoring the degree to which the foam disappeared after rubbing the foam ball between the hands.

Hair feel and shine were evaluated using straight brown hair strands with hair lengths of 17 cm (including 2 cm rubber coating at one end), widths of 2,5 cm and weights of 3 g.

Visual volume and curl definition were evaluated using thickly curled black hair strands with hair lengths of 23 cm (including 3 cm rubber coating at one end) and weights of 20 g. Hair strands were treated with the respective cosmetic compositions, applying the same quantity of product.

Hair feel was evaluated by running the hair between the fingers from root to end. If the hair ran through the fingers easily, a smooth feel was designated; if the hair was impeded from running through the fingers easily, this was referred to as a rough feel.

Visual volume was evaluated by assessing the volume of the hair.

Curl definition was evaluated by assessing the degree to which the curls were defined.

Hair shine was evaluated by looking at the reflection of light on the hair under standard conditions (box equipped with "Linestra fluorescent tube", adjustment: fluorescent illumination).

**Table 4**

| **sensory attribute** | **example invention** | **comparative example** |
|---|---|---|
| **usage experience:** | | |
| **foam extension** | 2,8 | 1.9 |
| (no foam extension - large foam extension) | | |
| **foam compactness** | 2,5 | 2,3 |
| (least - most compact) | | |
| **foam persistency** | 7.1 | 6,5 |
| (disappears quickly - stays long) | | |
| (least shiny - most shiny) | | |

| **hair performance:** | | |
|---|---|---|
| **hair feel** | 8,7 | 8,4 |
| (roughest - smoothest) | | |
| **visual volume** | 4,8 | 4,6 |
| (least volume - most volume) | | |
| **curl definition** | 6.6 | 6,0 |
| (least definition - most definition) | | |
| **shine** | 7,4 | 5,0 |

Results in table 4 clearly show that the composition according to the invention leads to both, improved usage experience and better hair performance with respect to the comparative example. According to the results in table 4, the example of the invention provides larger foam extension, more foam compactness, better foam persistency, i.e. the foam stays longer on the hands of a consumer, smoother hair feel, more visual volume of the hair, more curl definition and more hair shine with respect to the comparative example.

It is explicitly stated that all features disclosed in the description and/or the claims are intended to be disclosed separately and independently from each other for the purpose of original disclosure as well as for the purpose of restricting the claimed invention independent of the composition of the features in the embodiments and/or the claims. It is explicitly stated that all value ranges or indications of groups of entities disclose every possible intermediate value or intermediate entity for the purpose of original disclosure as well as for the purpose of restricting the claimed invention, in particular as limits of value ranges.

The dimensions and values disclosed herein are not to be understood as being strictly limited to the exact numerical values recited. Instead, unless otherwise specified, each such dimension is intended to mean both the recited value and a functionally equivalent range surrounding that value. For example, a dimension disclosed as "40 mm" is intended to mean "about 40 mm".

## Claims

1. Cosmetic composition in a container comprising at least one inner bag and an outer container, wherein the outer container encloses the inner bag and is filled with a propellant compressing the inner bag; and a valve mechanism attached to the inner bag moveable between an open position, in which a composition stored in the inner bag is allowed to be discharged by the pressure of the compressed gas as a foam, and a closed position, in which the composition is not allowed to be discharged, wherein the composition within the inner bag comprises
a) 0.1 to 5 % by weight of a cationic surfactant(A),
b) 0.1 to 10 % by weight of a fatty alcohol (B), and
c) 0.1 to 10 % by weight of carbon dioxide.

2. Composition according to claim 1, **characterized in that** it contains 70 to 98 % by weight of water.

3. Composition according to one of the preceding claims wherein the cationic surfactant (A) is selected from the group consisting of cetyl trimethyl ammonium salts, behenyl trimethyl ammonium salts, dimethyl ditallow ammonium salts and stearyl amido- propyl dimethylamine.

4. Composition according to one of the preceding claims, **characterized in that** it comprises at least one fatty alcohol having a melting point of more than 30°C and optionally at least one fatty alcohol having a melting point of 30°C or less, wherein the weight ratio of fatty alcohols having melting points to fatty alcohols having melting points of 30°C or lower is 0.25 or less in the case that the composition comprises at least one fatty alcohol having a melting point of more than 30°C and at least one fatty alcohol having a melting point of 30°C or less.

5. Composition according to one of the preceding claims, **characterized in that** the fatty alcohol with a melting point of 30°C or less is selected from the group consisting of unsaturated C12-C22 straight chain alcohols, saturated C12-C18 branched chain alcohols, saturated C8-C12 straight chain alcohols, and mixtures thereof.

6. Composition according to one of the preceding claims, **characterized in that** comprises a fatty alcohol having a melting point of 25°C or lower.

7. Composition according to one of the preceding claims, **characterized in that** it comprises from 0.1% to 10% by weight, of a hair conditioning agent selected from the group consisting of cationic polymers and nonvolatile non-crosslinked silicones, and mixtures thereof.

8. Composition according to one of the preceding claims, **characterized in that** the inner bag in the container has flat lateral edges.

9. Composition according to one of the preceding claims, **characterized in that** the inner bag comprises a bottom fold directed towards an upper end of the bag.

10. Composition according to one of the preceding claims, **characterized in that** the inner bag comprises flat triangular portions each extending from a bottom edge to the lateral edges with an angle of substantially 45 °.

11. Use of a container comprising at least one inner bag and an outer container, wherein the outer container encloses the inner bag and is filled with a propellant compressing the inner bag; and a valve mechanism attached to the inner bag moveable between an open position, in which a composition stored in the inner bag is allowed to be discharged by the pressure of the compressed gas in foam form, and a closed position, in which the composition is not allowed to be discharged, for discharging a cosmetic composition comprising
a) 0.1 to 5 % by weight of a cationic surfactant(A),
b) 0.1 to 10 % by weight of a fatty alcohol (B), and
c) 0.1 to 10 % by weight of carbon dioxide.

## Patentansprüche

1. Kosmetikzusammensetzung in einem Behälter, umfassend mindestens einen Innenbeutel und einen Außenbehälter, wobei der Außenbehälter den Innenbeutel einschließt und mit einem Treibstoff gefüllt ist, der den Innenbeutel komprimiert; und einen an dem Innenbeutel befestigten Ventilmechanismus, der zwischen einer offenen Position, in der eine in dem Innenbeutel aufbewahrte Zusammensetzung durch den Dnick des komprimierten Gases als Schaum abgegeben werden kann, und einer geschlossenen Position, in der die Zusammensetzung nicht abgegeben werden kann, beweglich ist, wobei die Zusanunensetzung in dem Innenbeutel
a) zu 0,1 bis 5 Gew.-% ein kationenaktives Tensid (A),
b) zu 0,1 bis 10 Gew.-% einen Fettalkohol (B) und
c) zu 0,1 bis 10 Gew.-% Kohlendioxid umfasst.

2. Zusammensetzung nach Anspruch 1, **dadurch gekennzeichnet, dass** sie 70 bis 98 Gew.-% Wasser enthält.

3. Zusammensetzung nach einem der vorstehenden Ansprüche, wobei das kationenaktive Tensid (A) ausgewählt ist aus der Gruppe bestehend aus Cetyltrimethylammoniumsalzen, Behenyltrimethylammoniumsalzen, Dimethylditalgammoniumsalzen und Stearylamidopropyldimetlrylamin.

4. Zusammensetzung nach einem der vorstehenden Ansprüche, **dadurch gekennzeichnet, dass** sie mindestens einen Fettalkohol mit einem Schmelzpunkt von mehr als 30 °C und wahlweise mindestens einen Fettalkohol mit einem Schmelzpunkt von 30 °C oder weniger umfasst, wobei das Gewichtsverhältnis von Fettalkoholen mit Schmelzpunkten zu Fettalkoholen mit Schmelzpunkten von 30 °C oder weniger 0,25 oder weniger beträgt, falls die Zusammensetzung mindestens einen Fettalkohol mit einem Schmelzpunkt von mehr als 30 °C und mindestens einen Fettalkohol mit einem Schmelzpunkt von 30 °C oder weniger umfasst.

5. Zusammensetzung nach einem der vorstehenden Ansprüche, **dadurch gekennzeichnet, dass** der Fettalkohol mit einem Schmelzpunkt von 30 °C oder weniger ausgewählt ist aus der Gruppe bestehend aus ungesättigten geradkettigen C12-C22-Alkoholen, gesättigten verzweigtkettigen C12-C18-Alkoholen, gesättigten geradkettigen C8-C12-Alkoholen und Mischungen davon.

6. Zusammensetzung nach einem der vorstehenden Ansprüche, **dadurch gekennzeichnet, dass** sie einen Fettalkohol mit einem Schmelzpunkt von 25 °C oder weniger umfasst.

7. Zusammensetzung nach einem der vorstehenden Ansprüche, **dadurch gekennzeichnet, dass** sie von 0,1 Gew.-% bis 10 Gew.-% ein Haarkonditioniermittel umfasst, ausgewählt aus der Gruppe bestehend aus kationischen Polymeren und nichtflüchtigen, nicht vernetzten Silikonen und Mischungen davon.

8. Zusammensetzung nach einem der vorstehenden Anspruche, **dadurch gekennzeichnet, dass** der Innenbeutel in dem Behälter flache Seitenränder aufweist.

9. Zusammensetzung nach einem der vorstehenden Ansprüche, **dadurch gekennzeichnet, dass** der Innenbeutel eine Bodenfaltung umfasst, die zu einem oberen Ende des Beutels hin ausgerichtet ist.

10. Zusammensetzung nach einem der vorstehenden Ansprüche, **dadurch gekennzeichnet, dass** der Innenbeutel flache dreieckige Abschnitte umfasst, die sich jeweils mit einem Winkel von im Wesentlichen 45° von einem unteren Rand zu den Seitenrändern erstrecken.

11. Verwendung eines Behälters, umfassend mindestens einen Innenbeutel und einen Außenbehälter, wobei der Außenbehälter den Innenbeutel einschließt und mit einem Treibstoff gefüllt ist, der den Innenbeutel komprimiert; und einen an dem Innenbeutel befestigten Ventilmechanismus, der zwischen einer offenen Position, in der eine in dem Innenbeutel aufbewahrte Zusammensetzung durch den Druck des komprimierten Gases in Schaumform abgegeben werden kann, und einer geschlossenen Position, in der die Zusammensetzung nicht abgegeben werden kann, beweglich ist, zum Abgeben einer Kosmetikzusammensetzung, umfassend
a) zu 0,1 bis 5 Gew.-% ein kationenaktives Tensid (A),
b) zu 0,1 bis 10 Gew.-% einen Fettalkohol (B) und
c) zu 0,1 bis 10 Gew.-% Kohlendioxid.

## Revendications

1. Composition cosmétique dans un récipient comprenant au moins un sac interne et un récipient externe, dans laquelle le récipient externe entoure le sac interne et est rempli avec un propulseur comprimant le sac interne ; et un mécanisme de soupape fixé au sac interne mobile entre une position ouverte, dans laquelle une composition stockée dans le sac interne peut être évacuée par la pression du gaz comprimé en tant que mousse, et une position fermée, dans laquelle la composition ne peut pas être évacuée, où la composition au sein du sac interne comprend
a) 0,1 à 5 % en poids d'un agent tensioactif cationique (A),
b) 0,1 à 10 % en poids d'un alcool gras (B), et
c) c) 0,1 à 10 % en poids de dioxyde de carbone.

2. Composition selon la revendication 1, **caractérisée en ce qu'**elle contient 70 à 98 % en poids d'eau.

3. Composition selon l'une des revendications précédentes, dans laquelle l'agent tensioactif cationique (A) est choisi dans le groupe constitué de sels de cétyltriméthylammonium, sels de béhényl-triméthylammonium, sels de diméthyl-disuif-ammonium et stéaryl-amido-propyl-diméthylamine.

4. Composition selon l'une des revendications précédentes, **caractérisée en ce qu'**elle comprend au moins un alcool gras possédant un point de fusion de plus de 30 °C et facultativement au moins un alcool gras possédant un point de fusion de 30 °C ou moins, dans laquelle le rapport pondéral d'alcools gras possédant des points de fusion sur les alcools gras possédant des points de fusion de 30 °C ou moins est 0,25 ou moins dans le cas où la composition comprend au moins un alcool gras possédant un point de fusion de plus de 30 °C et au moins un alcool gras possédant un point de fusion de 30 °C ou moins.

5. Composition selon l'une des revendications précédentes, **caractérisée en ce que** l'alcool gras avec un point de fusion de 30 °C ou moins est choisi dans le groupe constitué d'alcools insaturés à chaîne linéaire en C 12 à C22, d'alcools saturés à chaîne ramifiée en C12 à C18, d'alcools saturés à chaîne linéaire en C8 à C12, et leurs mélanges.

6. Composition selon l'une des revendications précédentes, **caractérisée en ce qu'**elle comprend un alcool gras possédant un point de fusion de 25 °C ou moins.

7. Composition selon l'une des revendications précédentes, **caractérisée en ce qu'**elle comprend de 0,1 % à 10 % en poids, d'un agent de conditionnement des cheveux choisi dans le groupe constitué de polymères cationiques et silicones non réticulées non volatiles, et leurs mélanges.

8. Composition selon l'une des revendications précédentes, **caractérisée en ce que** le sac interne dans le récipient possède des bords latéraux plats.

9. Composition selon l'une des revendications précédentes, **caractérisée en ce que** le sac interne comprend un pli inférieur dirigé vers l'extrémité supérieure du sac.

10. Composition selon l'une des revendications précédentes, **caractérisée en ce que** le sac interne comprend des parties triangulaires plates s'étendant chacune d'un bord inférieur jusqu'aux bords latéraux avec un angle essentiellement de 45°.

11. Utilisation d'un récipient comprenant au moins un sac interne et un récipient externe, dans laquelle le récipient externe entoure le sac interne et est rempli avec un propulseur comprimant le sac interne ; et un mécanisme de soupape fixé au sac interne mobile entre une position ouverte, dans laquelle une composition stockée dans le sac interne peut être évacuée par la pression du gaz comprimé sous forme de mousse, et une position fermée, dans laquelle la composition ne peut pas être évacuée, pour évacuer une composition cosmétique comprenant
a) 0,1 à 5 % en poids d'un agent tensioactif cationique (A),
b) 0,1 à 10 % en poids d'un alcool gras (B), et
c) 0,1 à 10 % en poids de dioxyde de carbone.
